# EUROPEAN PATENT SPECIFICATION

(11) **EP 2 777 713 B1**
(45) Date of publication and mention of the grant of the patent: **06.12.2017**
(21) Application number: 12848228.8
(22) Date of filing: 07.11.2012
(51) Int. Cl.: A61K 31/4745, A61P 35/00, A61K 47/50, A61K 9/02, A61K 9/20, A61K 9/08, A61K 9/12, A61K 9/48

(54) **POLYETHYLENE GLYCOL-AMINO ACID OLIGOPEPTIDE-IRINOTECAN DRUG CONJUGATE AND DRUG COMPOSITION THEREOF**
POLYETHYLENGLYCOL-AMINOSÄUREN- OLIGOPEPTID-IRINOTECAN-ARZNEIMITTELKONJUGAT UND ARZNEIMITTELZUSAMMENSETZUNG DARAUS
CONJUGUÉ MÉDICAMENTEUX DE POLYÉTHYLÈNEGLYCOL-OLIGOPEPTIDE D'ACIDES AMINÉS-IRINOTÉCAN ET COMPOSITION MÉDICAMENTEUSE DE CELUI-CI

(30) Priority: 07.11.2011 CN 201110348708
(43) Date of publication of application: 17.09.2014
(73) Proprietor: Jenkem Technology Co. Ltd. (Beijing), Beijing 100085 (CN); Jenkem Technology Co. Ltd. (Tianjin), Tianjin 300457 (CN)
(72) Inventor: XU, Lihua, Beijing 100192 (CN); HUANG, Wenzhe, Beijing 100192 (CN); ZHAO, Xuan, Beijing 100192 (CN)
(74) Representative: Hanna Moore + Curley
(86) International application number: PCT/CN2012/001498
(87) International publication number: WO 2013/067767

(56) References cited:
- WO-A1-03/076490
- CN-A- 1 442 440
- CN-A- 1 706 865
- CN-A- 1 706 865
- US-A1- 2005 147 617
- GUIOTTO ANDREA ET AL: "Synthesis, characterization, and preliminary in vivo tests of new poly(ethylene glycol) conjugates of the antitumor agent 10-amino-7-ethylcamptothecin", JOURNAL OF MEDICINAL CHEMISTRY, AMERICAN CHEMICAL SOCIETY, US, vol. 47, no. 5, 26 February 2004 (2004-02-26), pages 1280-1289, XP009121059, ISSN: 0022-2623, DOI: 10.1021/JM031072E

## Description

### Technical field

The present invention relates to the conjugates of polyethylene glycol, particularly the conjugate of polyethylene glycol with oligopeptides and drug irinotecan and their pharmaceutical compositions.

### Background of the invention

Irinotecan (CPT-11) is a derivative of camptothecin with good anti-cancer activities and unique mechanism of action. It is by far one of the best-selling anticancer drugs. Its worldwide sale in 2003 was more than $ 1 billion and had a 20% annual sales growth rate, thereby becoming one of the best-selling anti-cancer drugs in the world. Irinotecan has a very broad spectrum of anti-tumor. Results of stage I and II clinical studies showed that the drug had a positive effect against chemotherapy-resistant tumors such as metastatic colorectal cancer, non-small cell lung cancer, ovarian cancer and cervical cancer. Furthermore, it has a certain effect against gastric cancer, malignant lymphoma non-Hodgkin's lymphoma, breast cancer, small cell lung cancer, skin cancer and pancreatic cancer. Currently, it is mainly used as an effective drug for treatment of advanced colorectal cancer, even for those cases resistant to 5-FU. But irinotecan has significant toxic and side effects and low water solubility. The common adverse reactions include loss of appetite, nausea, vomiting, diarrhea, leukopenia and neutropenia, anemia and thrombocytopenia, alopecia and cholinergic syndrome. Of which, diarrhea is most frequent. Nearly 90% subjects underwent diarrhea, and nearly 30% of which underwent severe diarrhea. Therefore, a type of irinotecan with low toxicities is particularly required to improve the clinical antineoplastic effect by increasing the doses of irinotecan.

Polyethylene glycol (PEG) modification technology is a new drug delivery technology developed rapidly recently, it is mainly used in injection medication systems. It is a technology linking the activated polyethylene glycol to the drug molecule or surface. The PEG-drug derivatives obtained after PEG-modification will slowly release the small molecules of drug to achieve treatment efficacy. The small molecules of drug are mainly featured with the following advantages after PEGylation: 1. Increased water solubility; 2. Lower toxicity; 3. Extended circulating half-life of the drugs which reduce the frequency of drug administration, improve the patient compliance, improve the quality of life and reduce the cost of treatment; 4. Reduced enzymatic degradation which increase the bioavailability. After linking with PEG, the drug pharmacokinetics is changed; furthermore, the pharmacodynamics is changed. In particular, the PEG can maintain the blood drug at or close to the target concentration for a longer duration, thereby maintaining the full drug efficacy.

Currently, PEGylation technology has been widely used in the modification of the therapeutic grade proteins. Until the end of 2010, 4 of 6 the main types of PEG-modified drug products, namely PEG-intron®, PEGasys®, Neulasta® and Macugen® had the sales more than a hundred million of dollars. Furthermore, the sales of Neulasta® and Macugen® were more than one billion of dollars. MICERA (PEG-EPO produced by Roche) came into the market in Europe in the second half of 2007. Currently, dozens of PEGylated proteins drugs are in pre-clinical studies, and the PEGylated proteins drugs under clinical trials included superoxide dismutase produced by Enzon company (which will soon be on the market), interleukin-2 produced by Chiron Corporation (stage II) and etc. The applications of PEGylation technology in the medical field in the U.S. alone has created nearly $ 6 billion sales of new drugs and led to the booming development in the domain of new formulations. With the continuous successful research and development of a large number of new derivatives of PEG, PEGylation technology is not only used in modification of proteins, peptides and other macromolecular drugs, but also gradually used in the solubilization and attenuation of small molecules as well as improvement of the efficacy. Currently, PEGylated small molecule drugs have not yet been approved for marketing in the international market, but several products have entered phase II and III clinical trials.

The application of PEGylation technology in small molecule drugs is being developed rapidly. It is reported that PEG can be used to connect with many small molecule drugs. U.S. patent US5824701, US5840900 and Chinese patent CN1283643 reported the prodrugs produced by bonding of such derivatives with the paclitaxel. In such drug model, both ends of polyethylene glycol were only bonded to a taxol molecule. To improve the load capacity of drug molecules, patent US6153655 declared a structure of terminally-branched polyethylene glycol. Two functional groups were generated by linking the structure to both ends of polyethylene glycol through amino groups. However, the introduction of non-biological branched small molecule also led to uncertainty of the drugs. Patents US5977163, US6262107 and CN1164533 disclosed a PGA-supported paclitaxel prodrug. In this drug model, the paclitaxels were randomly connected to the active carboxyl group of glutamic acid along the backbone chain of polyglutamate. Relatively wide distribution of polymerization rate and the uncertainty of the toxicity of poly-glutamic acid limited the applications of the invention. U.S. patent US7744861 disclosed a class of structures with multi-branched PEG binding to irinotecan. However, in these structures, each terminal group of the polyethylene glycol was only connected with one irinotecan molecule, thereby limiting the drug-loading capacity.

Beijing Jenkem technology Co., Ltd. began the researches on PEGylation of small molecule drug as early as 2002 and a number of patents had been registered (ZL03 8 01109.3, ZL 2004 1 0029615.3, ZL 2004 8 0005763.X, ZL 2008 1 0093688.7, ZL 02 1 07842.4 and ZL 02 1 08778.4). Of which, the "conjugate of hydrophilic polymer-polycarboxyl oligopeptide with drug molecule, the composition containing the conjugate and the usage" (ZL 02106691.4) provides a PEG derivative linked to multi-carboxy oligopeptide and suitable to link with multiple small molecule drugs.

The invention is based on the previous work, particularly the previous invention patent (ZL 2004 1 0048016.6) of our laboratory. ZL 200410048016.6 discloses a branching polyethylene glycol-oligopeptide, and drug conjugate comprising the same to reduce the drug dosage and avoid some toxic and side effect through improving the load factor of drug molecule; wherein the PEG can be a molecular weight of 300 to 60,000 Daltons, and the molecular weight of PEG polymer are all 10,000 Daltons among the embodiment 1-15 disclosed in the description, and ZL 200410048016.6 further discloses that the branching structure is conducive to carrying more drug molecules, which explicitly indicates that as compared to straight chain PEG-pentapeptides, the branching PEG-pentapeptides may be used to carry more drug molecules, and teaches a skilled person away from using a drug conjugate with straight-chain PEG portion. The inventors of the present invention adopt the PEG having a straight-chain structure and a molecular weight of 20,000-40,000 Daltons, and the oligopeptide consisting of 2 glutamic acid and 3 glycine residues, or 3 glutamic acid and 4 glycine residues to provide a novel PEG-amino acid oligopeptide-irinotecan conjugate.

The inventors of the present application solve the problems such as low water solubility and toxic side effects of irinotecan using PEGylation technique. The provided novel PEG- amino acid oligopeptide-irinotecan conjugate has good water solubility and can achieve the purpose of slow release through the regulation of the enzyme in the human body. It has a long duration of efficacy. Its anti-tumor activity is equivalent and even superior to that of irinotecan. Since the anti-tumor effect of irinotecan is dose-dependant, low toxicity PEGylated irinotecans can further improve the clinical anti-tumor effect of irinotecans by increasing the dose.

### Summary of the invention

On one hand, the present invention provides a conjugate of PEG-oligopeptide-irinotecan with the general formula (I): 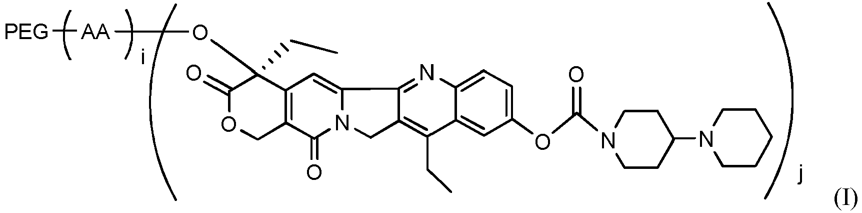 Wherein:
PEG represents polyethylene glycol;
(AA)ᵢ represents oligopeptide;
j is an integer of 2-12 representing the number of irinotecan linked with oligopeptide.

The PEG has an average molecular weight of 20,000 to 40,000 daltons. The PEG has a straight chain structure.

In the embodiments of the present invention, PEG is a linear methoxy-PEG with the following structure: 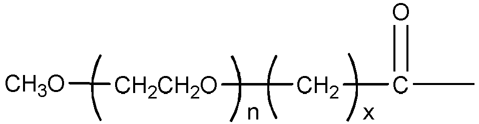 Wherein:
n is an integer of 400-500;
x is an integer of 0-6 , the preferable x is 1.

The amino acids oligopeptide consists of two glutamic acids and three glycines. In additional implementation protocols of the present invention, the described amino acids are the combination of glutamic acid and glycine which consists of three glutamic acids and four glycines.

On the other hand, the present invention provides the pharmaceutical compositions comprising the above described conjugates and the pharmaceutically acceptable carrier or excipient. The described pharmaceutical compositions are dosage forms of tablet, suppository, pill, soft and hard gelatin capsule, powder, solutions, suspension and aerosol. The described pharmaceutical compositions have anticancer activity, specifically including inhibitory activity against colon cancer, ovarian cancer and lung cancer.

The present invention provides a modification through the hydrophilic polymer to protect the irinotecan, thereby offering a modified irinotecan drug with improved drug absorption, prolonged duration of action, enhanced efficacy, reduced the dose as well as low toxic and side effects.

### Mode for carrying out the invention

Irinotecan is a camptothecin compound with a structure shown in formula (III). 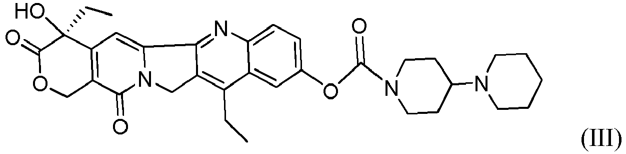 Structure of irinotecan

The structure of irinotecan contains a hydroxyl group, which can bind with polymer which has undergone terminal group modification through the ester group, carbonate group, amide group and other modes to achieve the effective protection and rational utilization of the irinotecan molecules. In particular, the ester groups can release active ingredients of the drug in the organism through biodegradation. Pharmaceutical formulations modified by this method are highly water-soluble, fast-working, durable and effective in cancer treatment.

Conjugates involved in the present invention is prepared as follows: the hydrophilic polymer PEG is modified by introducing reactive functional group carboxyl which is linked to an oligopeptide, meanwhile, the hydroxyl group on the irinotecan react with carboxyl group of glycine to form esters, then the carboxyl groups on the oligopeptide and combine with amino group of glycine on the irinotecan. This reaction mode not only ensures the in vivo activity of irinotecan, but also maximizes the yield and purity of the reaction product.

The structure of straight-chain PEG-carboxylic acid is shown in formula (IV): 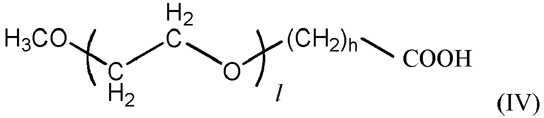 The structure formula of straight-chain PEG-carboxylic acid

The PEG is commonly represented as molecular weight, n is about 450, which corresponds to a molecular weight of 20,000 for PEG.

The oligopeptides in the present invention do not have straight-chain structures, the amide linkages in the oligopeptides are formed on the side chains of certain amino acids. The oligopeptides are glutamic acid and glycine.

The conjugates of the invention may be administered in the form of pure compound or appropriate pharmaceutical composition by any acceptable routes of administration or reagents used in similar usage. Therefore, the adoptable routes of administration include oral, intranasal, parenteral, topical, transdermal or rectal paths. The forms include solid, semi-solid, lyophilized powder, or liquid preparation, such as tablets, suppositories, pills, soft and hard gelatin capsules, powders, solutions, suspension and aerosols, and the dosage unit form suitable for simple administration with precise dosage is preferred. The compositions may contain conventional pharmaceutical carriers or excipient and the conjugates of the present invention functioning as active ingredients (one or more types), as well as other agents, carriers, adjuvants, and etc.

Typically, depending on the desired mode of administration, the pharmaceutically acceptable composition contains the conjugate of this invention of about 1% to 99% in weight and suitable pharmaceutical excipients of 99% to 1% in weight. Preferably, the composition contains the conjugate of the present invention of about 5 % to 75% by weight, and the rest is suitable pharmaceutical excipient.

The pharmaceutical compositions can be administered in the form of liquid. Specifically, the conjugate of the present invention (about 0.5% to 20%) and the selected pharmaceutical adjuvant can be dissolved and dispersed in the carriers such as water, saline, dextrose hydrate, glycerol and ethanol in practice to form a solution or suspension.

If indicated, small amounts of adjunct such as wetting agent or emulsifying agent, pH buffering agent and antioxidant, namely citric acid, sorbitan monolaurate, triethanolamine oleate butylated hydroxytoluene, and etc. may be contained in the pharmaceutical composition of the present invention.

The following embodiments are used to illustrate the invention but are not used to limit the invention.

### Embodiments

The PEG-glutamic acid dipeptide used in the embodiments are provided by Beijing JenKem technology Co., Ltd; irinotecans are purchased from Shanghai Longxiang Biomedical Development Co. Ltd; L(+)-glutamic acids are purchased from Beijing Chemical Reagent Company; p-toluene sulfonic acid, benzyl alcohol and dicyclohexylcarbodiimide (DCC) are purchased from Chemical reagent Co., Ltd. of Sinopharm Group Corporation; 4-dimethylaminopyridine (DMAP) and 1-hydroxybenzotriazole triazole (HOBT) are purchased form from Shanghai covalent chemical technology Co., Ltd.; N-(tert-butyloxycarbonyl) glycine are purchased from Sichuan Tongsheng Amino Acid Co. Ltd.; N-(tert-butyloxycarbonyl) glycine are purchased from Chengdu Jingtian Biology Co., Ltd..

### Embodiment 1: Preparation of conjugate of straight-chain PEG (20k)-pentapeptide-irinotecan (CPT-1)

L(+)-glutamic acid 29.4 g, p-toluene sulfonic acid 40 g and 80 ml benzyl alcohol were dissolved in 500 ml of toluene, the solutions were refluxed under nitrogen and 11 ml water was separated, the reflux was continued for 3 h and 150 ml water was evaporated. The solutions were cooled to 50 °C, the reaction solutions were poured into a beaker containing 600 ml petroleum ether and stirred for 1 h, and the precipitate was collected by filtration. The filter cake was dissolved in 280 ml 95% ethanol by heating and allowed to cool overnight. The precipitate was collected by filtration and dried under vacuum, then 61 g L (+)-glutamic acid dibenzyl ester p-toluenesulfonate was prepared.

30 g glutamic acid dibenzyl ester p-toluenesulfonate was dissolved in 500 ml dichloromethane, to which 20.86 g t-butyloxycarbonyl-L-glutamic acid-5-benzyl ester was added under nitrogen, 7.55 g 4-dimethyl-amino pyridine (DMAP) and 8.35 g 1-hydroxybenzotriazole (HOBT) were added after the above reagents were fully dissolved, then 40 ml dichloromethane with 14.3 g dicyclohexylcarbodiimide (DCC) was added after recomplete dissolution of the previously added reagents. After the titration was completed, the reaction system was closed and allowed to react overnight. Completion of the reaction was monitored by TLC. After the solvents were removed by filtration, 20 ml ethyl acetate was added to the concentrated solution, the solids were then filtered and 400 ml petroleum ether was added to the mother liquor for precipitation. Finally, 15.8 g reaction product Boc-Glu (obzl)-Glu (obzl)-obzl was obtained by filtration.

0.776 g Boc-Glu (obzl)-Glu (obzl)-obzl was dissolved in 7 ml dichloromethane, and 3 ml trifluoroacetic acid was added before the solution was allowed to react at room temperature for 2 h. The solvent was removed and 100 ml dichloromethane was added, and then 5% sodium bicarbonate solution (NaHCO3) was used to adjust the pH value to 7-8. Extraction and skimming were performed; the organic phase was washed twice with 5% sodium bicarbonate solution (NaHCO₃) and dried with anhydrous sodium sulfate. The dried product was filtered, and the filtrate was added directly to the reaction flask, 20.0 g PEG-acetic acid (molecular weight 20000), 245 mg 4-dimethylaminopyridine (DMAP) and 135 mg 1-hydroxybenzotriazole (HOBT) were added under nitrogen protection. After all the reagents were dissolved, 412 mg dicyclohexylcarbodiimide (DCC) was added. The solution was stirred for reaction at room temperature overnight. The solid substances were filtered, the redundant solvent was removed by rotary evaporation, 500 ml of isopropyl alcohol (IPA) was added to the residue, and the product was obtained by filtration and dried under vacuum. The dried product was dissolved in 200 ml anhydrous methanol, and then 1.0 g palladium-carbon was added, the hydrogen (H2) was ventilated into the solution to react at room temperature overnight. The palladium carbon was removed by filtration and the redundant solvent was removed by rotary evaporation, 500 ml isopropyl alcohol (IPA) was added to the residue, the product was obtained by filtration and dried under vacuum. Finally, 13.4 g PEG-Glu dipeptide (molecular weight 20000) was obtained.

13.3 g irinotecan hydrochloride and 20.1 g N-(tert-butyloxycarbonyl)-glycine (BOC-gly-OH) was dissolved in 120 ml anhydrous dichloromethane, and then 18.8 g dicyclohexyl carbodiimide (DCC) and7.4 g 4-dimethylaminopyridine (DMAP) were added to the solution, the reaction system was stirred at room temperature overnight. The solid reaction product was filtered off and the solution was concentrated under reduced pressure, 100 ml petroleum ether was added to the solution. The precipitate was collected by filtration and dried under vacuum, thus 23 g irinotecan-N-tert-butoxycarbonyl glycine ester was prepared. 23 g irinotecan -N-tert-butoxycarbonyl glycinate obtained from the previous step was dissolved in 100 ml dichloromethane which was then added with 30 ml trifluoroacetic acid, the solution was stirred at room temperature for 5 hours and concentrated under reduced pressure before addition of 500 ml diethyl ether. The reaction product was filtered and the precipitate was collected and dried under vacuum, and thus 20 g irinotecan-glycinate was prepared.

5.0 g PEG-glutamic acid dipeptide (molecular weight 20000), 1.02 g irinotecan-glycinate, 115 mg N-hydroxysuccinimide (NHS) and 153 mg 4- dimethylaminopyridine (DMAP) was dissolved in 50 ml anhydrous dichloromethane, under nitrogen protection, the solution was then added with 309 mg dicyclohexylcarbodiimide (DCC). The reaction system was stirred at room temperature overnight. The solid substances were filtered off and redundant solvent was removed by rotary evaporation, and then the residue was added with 100 ml isopropyl alcohol (IPA), the product was obtained by filtration and dried under vacuum, and thus 4.4 g PEG-Glu-Gly pentapeptide-irinotecan (CPT-1) was obtained. The melting point was 56 to 64 °C. 1H-NMR (DMSO-d6): 0.84-0.89 (m, 8H), 1.24 (m, 9H), 1.40-1.50 (m, 21H), 1.79 (m, 8H), 2.12 (m, 10H), 2.89 (m, 4H), 3.50 (m, 1800H), 4.05 (m, 9H), 4.26 (m, 5H), 5.26 (m, 5H), 5.48 (m, 5H), 7.07 (m, 2H), 7.55-7.60 (m, 3H), 7.89 (m, 3H), 8.11 (m, 4H), 8.20 (m, 3H).

### Embodiment 2: Preparation of straight-chain PEG-acetic acid (20k)-heptapeptide-irinotecan conjugate

L(+)-glutamic acid 29.4 g, p-toluene sulfonic acid 40 g and 80 ml benzyl alcohol were dissolved in 500 ml of toluene, the solutions were refluxed under nitrogen and 11 ml water was separated, the reflux was continued for 3 h and 150 ml water was evaporated. The solutions were cooled to 50 °C, the reaction solutions were poured into a beaker containing 600 ml petroleum ether and stirred for 1 h, and the precipitate was collected by filtration. The filter cake was dissolved in 280 ml 95% ethanol by heating and allowed to cool overnight. The precipitate was collected by filtration and dried under vacuum, then 61 g L (+)-glutamic acid dibenzyl ester p-toluenesulfonate was prepared.

30 g glutamic acid dibenzyl ester p-toluenesulfonate was dissolved in 500 ml dichloromethane, to which 20.86 g t-butyloxycarbonyl-L-glutamic acid-5-benzyl ester was added under nitrogen, 7.55 g 4-dimethyl-amino pyridine (DMAP) and 8.35 g 1-hydroxybenzotriazole (HOBT) were added after the above reagents were fully dissolved, then 40 ml dichloromethane with 14.3 g dicyclohexylcarbodiimide (DCC) was added after recomplete dissolution of the previously added reagents. After the titration was completed, the reaction system was closed and allowed to react overnight. Completion of the reaction was monitored by TLC. After the solvents were removed by filtration, 20 ml ethyl acetate was added to the concentrated solution, the solids were then filtered and 400 ml petroleum ether was added to the mother liquor for precipitation. Finally, 15.8 g reaction product Boc-Glu (obzl)-Glu (obzl)-obzl was obtained by filtration.

6.47 g Boc-Glu(obzl)-Glu (obzl)-obzl was dissolved in 15 ml dichloromethane, and 6 ml trifluoroacetic acid was added before the solution was allowed to react at room temperature for 2 h. The solvent was removed and 100 ml dichloromethane was added, and then 5% sodium bicarbonate solution (NaHCO3) was used to adjust the pH value to 7-8. Extraction and skimming were performed, and the organic phase was washed twice with 5% sodium bicarbonate solution (NaHCO₃) and dried with anhydrous sodium sulfate. The dried product was filtered and the filtrate was added directly to the reaction flask, 3.37 g Boc-L-Glutamic acid 5-benzyl ester was added under nitrogen protection. After all the reagents were dissolved, 1.22 g 4-dimethylaminopyridine (DMAP) and 1.35 g 1-hydroxybenzotriazole (HOBT) were added, after the reagents were fully dissolved, 20 ml dichloromethane containing 2.39 g dicyclohexylcarbodiimide (DCC) was added to the solution. After the titration was completed, the reaction system was closed and allowed to react overnight. Completion of the reaction was monitored by TLC. The filtered reaction product was extracted using 30 ml 10% citric acid thrice, 5% sodium hydrogen carbonate and 5% saturated aqueous sodium chloride three times in turn, and then dried using anhydrous sodium sulfate. After the product was filtered to remove the solvent, 25 ml ethyl acetate was added into the concentrated solution. The solid substances were filtered and 400 mlpetroleum ether was added into the mother liquor for precipitation. The 6.8 g product of Boc-Glu (obzl)-Glu (obzl) - Glu (obzl)-obzl was obtained after filtration.

1.039 g Boc-Glu (obzl)-Glu (obzl)-Glu (obzl)-obzl was dissolved in 7 ml dichloromethane, and 3 ml trifluoroacetic acid was added before the solution was allowed to react at room temperature for 2 h. The solvent was removed and 200 ml dichloromethane was added, and then 5% sodium bicarbonate solution (NaHCO3) was used to adjust the pH value to 7-8. Extraction and skimming were performed; the organic phase was washed twice with 5% sodium bicarbonate solution (NaHCO₃) and dried with anhydrous sodium sulfate. The dried product was filtered, and the filtrate was added directly to the reaction flask, 20.0 g PEG-acetic acid (molecular weight 20000), 245 mg 4-dimethylaminopyridine (DMAP) and 135 mg 1-hydroxybenzotriazole (HOBT) were added under nitrogen protection. After all the reagents were dissolved, 412 mg dicyclohexylcarbodiimide (DCC) was added. The solution was stirred for reaction at room temperature overnight. The solid substances were filtered, the redundant solvent was removed by rotary evaporation, 500 ml of isopropyl alcohol (IPA) was added to the residue, and the product was obtained by filtration and dried under vacuum. The dried product was dissolved in 200 ml anhydrous methanol, and then 1.0 g palladium carbon was added, the hydrogen (H2) was ventilated into the solution to react at room temperature overnight. The palladium carbon was removed by filtration and the redundant solvent was removed by rotary evaporation, 500 ml isopropyl alcohol (IPA) was added to the residue, the product was obtained by filtration and dried under vacuum. Finally, 15.4 g PEG-Glu-tripeptide (molecular weight 20000) was obtained.

5.0 g PEG-Gly-tripeptide (prepared in the present embodiment, the molecular weight was 20000), 1.36 g irinotecan-glycinate (prepared in embodiment 1), 115 mg N-hydroxysuccinimide (NHS) and 153 mg 4-dimethylaminopyridine (DMAP) were dissolved in 50 ml anhydrous dichloromethane, and then 309 mg dicyclohexylcarbodiimide (DCC) was added under nitrogen protection. The reaction system was stirred at room temperature overnight. The solid product was filtered off and the redundant solvent was removed by rotary evaporation. The residue was mixed with 100 ml isopropyl alcohol (IPA), and then the solution was filtered and the product was dried under vacuum. Thus, 4.5 g straight-chain PEG-Glu-Gly-heptapeptide-irinotecan was obtained. The melting point was 57-64 °C. 1H-NMR (DMSO-d6): 0.84-0.89 (m, 8H), 1.24 (m, 9H), 1.71-1.74 (m, 23H), 2.12 (m, 16H), 2.95-2.99 (m, 19H), 3.50 (m, 1800H), 4.22 (m, 18H), 5.45 (m, 5H), 5.49 (m, 5H), 7.07 (m, 2H), 7.55-7.60 (m, 3H), 7.89 (m, 3H), 8.11 (m, 4H), 8.20 (m, 3H).

### Embodiment 3: The inhibitory effect of various irinotecan preparations on transplanted tumors of human colon cancer cells HCT-116 in nude mice

### Test substances

CPT-1 was a straight-chain PEG-acetic acid-pentapeptides-irinotecan conjugate.

### Drug used as positive control

40 mg/2 ml irinotecan hydrochloride injection (CPT-11) produced by the Aventis Pharma (Dagenham) company, lot number 8UL002-B. The drug was diluted with saline to the desired concentration just before utilization.

### Dose and administration

CPT-1 dose was set as 45 mg/kg (content of irinotecan), which was administered intravenously once a week for three consecutive weeks; the CPT-11 was intravenously administrated once a week at 45mg/kg and intravenously administered three times a week for three consecutive weeks at 15 mg/kg.

### Animals

BALB/cA nude mice (male, 4-6 weeks old, weighed 19±2 g) were provided by the Shanghai Institute of Materia Medica, Chinese Academy of Sciences. The production certificate number was SCXK (Shanghai) 2008-0017. Animal numbers of each group: 12 mice in negative control group and 6 mice in treatment group.

### Cell lines

Human colon tumor cell line HCT-116 was purchased from ATCC. 5 x 10⁶ cells were subcutaneously inoculated in the right armpit of each nude mouse. The transplanted tumors were transferred in nude mice for two generations before utilization.

### Experimental methods

Vigorous tumor tissues were cut into pieces of about 1.5 mm3 and then subcutaneously inoculated to the right armpit nude mice under sterile conditions. The diameters of the subcutaneous transplanted tumors were measured using a vernier caliper. The animals were randomly grouped when the tumors grew to 100-200 mm3.

The animals in treatment group and control group were intravenously administrated with 45 mg/kg CPT-1 and equivalent saline respectively once a week for three consecutive weeks. CPT-11 (15 mg/kg) was intravenously administrated as a positive control drug three times a week for consecutive three weeks. After the administration, the animals were observed consecutively for one week. During the whole experiment, diameters of the transplanted tumors were measured and the animals were weighed two times a week. The tumor volume (TV) was calculated as follows: TV= 1/2 x a x b², wherein, a and b represented length and width respectively. According to the results of measurements, the relative tumor volumes (RTV) were calculated as follows: RTV=Vt/V0. Wherein, V0 were the volumes measured at administration after caging (i.e. d0), Vt were the tumor volumes of every measurements. The index used in evaluation of anti-tumor activity was relative tumor proliferation rate T/C (%) calculated as follows: T/C (%) = (TRTV/CRTV) x 100%, TRTV was RTV of treatment group, CRTV was RTV of negative control group.

### Results and discussion

The results were shown in Table 1. CPT1 (45 mg/kg) administered intravenously once weekly for three consecutive weeks significantly inhibited the growth of subcutaneous transplanted tumors of human colon cancer cell line HCT-116 cells in nude mice, the T/C % was 27.60%, and the inhibitory effects were superior to those of CPT-11 administered with the same dose and regimen. The experimental treatment using CPT-11 administrated with the same regimen also inhibited the growth of subcutaneous transplanted tumors of HCT-116 cells, but the T/C % was only 63.56%. The positive control CPT-11 intravenously administrated at 15 mg/kg three times a week for three consecutive weeks could also significantly inhibited the growth of subcutaneous transplanted tumors of HCT-116 cells with a T/C value of 39.84%. During the whole experiment, nude mice in each treatment group underwent weight loss. Only nude mice in 45 mg/kg CPT-11 group underwent slightly higher weight loss, compared with those in solvent control group.

**Table 1 Experimental treatment effects of PEGylated irinotecan on transplanted tumors of human colon cancer cells HCT-116 in nude mice**

| Group | Dose and administration | | Number of animals | | Weight (g) | | TV(mm³, mean±SD) | | RTV(mean±SD) | T/C (%) |
|---|---|---|---|---|---|---|---|---|---|---|
| | | | At the beginning | At the beginning | At the beginni ng | At lats | d₀ | d₂₈ | | |
| Solvent control | 0.4ml/mice qw×3w | iv | 12 | 12 | 19.7 | 17.3 | 132±32 | 804±82 | 6.45±1.83 | |
| CPT-11 | 15mg/kg, q3w×3w | iv | 6 | 6 | 19.1 | 16.6 | 129±26 | 322±37 | 2.57±0.66** | 39.84 |
| CPT-11 | 45mg/kg, qw×3w | iv | 6 | 6 | 19.2 | 15.1 | 126±25 | 512±99 | 4.10±0.73* | 63.56 |
| CPT-1 | 45mg/kg, qw×3w | iv | 6 | 6 | 18.8 | 16.8 | 132±30 | 234±54 | 1.78±0.23**^{,##} | 27.60 |

| | | | | | | | | | | |
|---|---|---|---|---|---|---|---|---|---|---|
| Note: t test, vs. solvent control group, * p <0.01, ** p <0.001; *vs*. 45mg/kg CPT-11 group, # p <0.001 | | | | | | | | | | |

### Embodiment 4: The inhibitory effect of various irinotecan preparations on transplanted tumors of human colon cancer cells HT-29 in nude mice

### Test substances

CPT-1 was a straight-chain PEG-acetic acid-pentapeptides-irinotecan conjugate.

### Drug used as positive control

40 mg/2 ml irinotecan hydrochloride injection (CPT-11) produced by the Aventis Pharma (Dagenham) company, lot number 8UL002-B. The drug was diluted with saline to the desired concentration just before utilization.

### Dose and administration

CPT-1 dose was set as 45 mg/kg (content of irinotecan), which was administered intravenously once a week for three consecutive weeks; the CPT-11 was intravenously administrated once a week at 45 mg/kg and intravenously administered three times a week for three consecutive weeks at 15 mg/kg.

### Animals

BALB/cA nude mice (male, 5-6 weeks old, weighed 18±2 g) were provided by the Shanghai Institute of Materia Medica, Chinese Academy of Sciences. The production certificate number was SCXK (Shanghai) 2008-0017. Animal numbers of each group: 12 mice in negative control group and 6 mice in treatment group.

### Cell lines

Human colon tumor cell strains HT-29 were purchased from ATCC. 5 x 10⁶ cells were subcutaneously inoculated in the right armpit of each nude mouse. The transplanted tumors were transferred in nude mice for two generations before utilization.

### Experimental methods

Vigorous tumor tissues were cut into pieces of about 1.5 mm3 and then subcutaneously inoculated to the right armpit nude mice under sterile conditions. The diameters of the subcutaneous transplanted tumors were measured using a vernier caliper. The animals were randomly grouped when the tumors grew to 100-200 mm3.

The animals in treatment group and control group were intravenously administrated with 45 mg/kg CPT-1 and equivalent saline respectively once a week for three consecutive weeks. CPT-11 (15 mg/kg) was intravenously administrated as a positive control drug three times a week for consecutive three weeks. After the administration, the animals were observed consecutively for one week. During the whole experiment, diameters of the transplanted tumors were measured and the animals were weighed two times a week. The tumor volume (TV) was calculated as follows: TV= 1/2 x a x b², wherein, a and b represented length and width respectively. According to the results of measurements, the relative tumor volumes (RTV) were calculated as follows: RTV=Vt/V0. Wherein, V0 were the volumes measured at administration after caging (i.e. d0), Vt were the tumor volumes of every measurements. The index used in evaluation of anti-tumor activity was relative tumor proliferation rate T/C (%) calculated as follows: T/C (%) = (TRTV/CRTV) x 100%, TRTV was RTV of treatment group, CRTV was RTV of negative control group.

### Results and discussion

The results were shown in Table 2. CPT1 (45 mg/kg) administered intravenously once weekly for three consecutive weeks significantly inhibited the growth of subcutaneous transplanted tumors of human colon tumor cell line HT-29 cells in nude mice. The growth rate of the tumor in the mice of experimental group was reduced one week after administration. The T/C % was 41.08% when the experiment was completed. The positive control CPT-11 intravenously administrated at 15 mg/kg three times a week for three consecutive weeks could also significantly inhibited the growth of subcutaneous transplanted tumors of HT-29 cells with a T/C value of 27.27%. During the whole experiment, nude mice in each treatment group had good growing conditions; only nude mice in two groups treated with different doses of CPT-11 underwent weight loss.

**Table 2. Experimental treatment effects of PEGylated irinotecan on transplanted tumors of human colon caner cells HT-29 in nude mice**

| Group | Dose and administration | | Number of animals | | Weight (g) | | TV(mm³, mean±SD) | | RTV (mean±SD) | T/C (%) |
|---|---|---|---|---|---|---|---|---|---|---|
| | | | At the beginning | At the beginning | At the beginning | At lats | d₀ | d₂₈ | | |
| Solvent control | 0.4ml/mice qw×3w | iv | 12 | 12 | 19.0 | 19.2 | 119±29 | 1588± 578 | 13.90± 5.38 | |
| CPT-11 | 15mg/kg, q3w×3w | iv | 6 | 6 | 18.2 | 16.0 | 121±14 | 460± 152 | 3.79± 1.21*^{,} | 27.27 |
| CPT-11 | 45mg/kg, qw×3w | iv | 6 | 6 | 18.6 | 16.2 | 116±13 | 995± 368 | 8.69± 3.43*** | 62.52 |
| CPT-1 | 45mg/kg, qw×3w | iv | 6 | 6 | 18.7 | 18.4 | 115±25 | 642± 105 | 5.71± 1.21** | 41.08 |

| | | | | | | | | | | |
|---|---|---|---|---|---|---|---|---|---|---|
| Note: t test, *vs*. solvent control group, * p <0.05, ** p <0.01, *** p <0.01; *vs*. 45mg/kg CPT-11 group, # p <0.05 | | | | | | | | | | |

### Embodiment 5: The inhibitory effect of various irinotecan preparations on transplanted tumors of human lung cancer cells A549 in nude mice

### Test substances

CPT-1 was a straight-chain PEG-acetic acid-pentapeptides-irinotecan conjugate.

### Drug used as positive control

40 mg/2 ml irinotecan hydrochloride injection (CPT-11) produced by the Aventis Pharma (Dagenham) company, lot number 8UL002-B. The drug was diluted with saline to the desired concentration just before utilization.

### Dose and administration

CPT-1 dose was set as 45 mg/kg (content of irinotecan), which was administered intravenously once a week for three consecutive weeks; the CPT-11 was intravenously administrated once a week at 45 mg/kg and intravenously administered three times a week for three consecutive weeks at 15 mg/kg.

### Animals

BALB/cA nude mice (male, 5-6 weeks old, weighed 18±2 g) were provided by the Shanghai Institute of Materia Medica, Chinese Academy of Sciences. The production certificate number was SCXK (Shanghai) 2008-0017. Animal numbers of each group: 12 mice in negative control group and 6 mice in treatment group.

### Cell lines

Human lung tumor cell strains A549 were purchased from ATCC. 5 x 10⁶ cells were subcutaneously inoculated in the right armpit of each nude mouse. The transplanted tumors were transferred in nude mice for two generations before utilization.

### Experimental methods

Vigorous tumor tissues were cut into pieces of about 1.5 mm3 and then subcutaneously inoculated to the right armpit nude mice under sterile conditions. The diameters of the subcutaneous transplanted tumors were measured using a vernier caliper. The animals were randomly grouped when the tumors grew to 100-200 mm3.

The animals in treatment group and control group were intravenously administrated with 45 mg/kg CPT-1 and equivalent saline respectively once a week for three consecutive weeks. CPT-11 (15 mg/kg) was intravenously administrated as a positive control drug three times a week for consecutive three weeks. After the administration, the animals were observed consecutively for one week. During the whole experiment, diameters of the transplanted tumors were measured and the animals were weighed two times a week. The tumor volume (TV) was calculated as follows: TV= 1/2 x a x b², wherein, a and b represented length and width respectively. According to the results of measurements, the relative tumor volumes (RTV) were calculated as follows: RTV=Vt/V0. Wherein, V0 were the volumes measured at administration after caging (i.e. d0), Vt were the tumor volumes of every measurements. The index used in evaluation of anti-tumor activity was relative tumor proliferation rate T/C (%) calculated as follows: T/C (%) = (TRTV/CRTV) x 100%, TRTV was RTV of treatment group, CRTV was RTV of negative control group.

### Results and discussion

The results were shown in Table 3. CPT1 (45 mg/kg) administered intravenously once weekly for three consecutive weeks significantly inhibited the growth of subcutaneous transplanted tumors of human lung cancer cell line A549 cells in nude mice, the T/C % was 20.62%, and the inhibitory effects were superior to those of CPT-11 administered with the same dose and regimen. The growth rate of the subcutaneous transplanted tumors in tumor bearing mice was reduced one week after the experimental treatment using CPT-1. The positive control CPT-11 intravenously administrated at 15 mg/kg three times a week for three consecutive weeks could also significantly inhibited the growth of subcutaneous transplanted tumors of A549 cells with a T/C value of 53.26%. During the whole experiment, nude mice in each treatment group had good growing conditions and slightly lower weight gain, compared with the mice in solvent control group.

**Table 3. Experimental treatment effects of PEGylated irinotecan on transplanted tumors of human lung cancer cells A549 in nude mice**

| Group | Dose and administration | | Number of animals | | Weight (g) | | TV (mm³, mean±SD) | | RTV (mean±SD) | T/C (%) |
|---|---|---|---|---|---|---|---|---|---|---|
| | | | At the beginning | At the beginning | At the beginning | At lats | d₀ | d₂₈ | | |
| Solvent control | 0.4ml/mice qw×3w | iv | 12 | 12 | 18.4 | 23.1 | 108+21 | 1546± 496 | 14.40± 4.38 | |
| CPT-11 | 15mg/kg, q3w×3w | iv | 6 | 6 | 18.1 | 21.2 | 112+21 | 831± 305 | 7.67± 2.88**^{,} | 53.26 |
| CPT-11 | 45mg/kg, qw×3w | iv | 6 | 6 | 18.4 | 21.7 | 110±15 | 1055+ 432 | 9.87± 4.87 | 68.54 |
| CPT-1 | 45mg/kg, qw×3w | iv | 6 | 6 | 19.1 | 22.0 | 105+21 | 320+ 154 | 2.97±1.04*^{,#} | 20.62 |

| | | | | | | | | | | |
|---|---|---|---|---|---|---|---|---|---|---|
| Note: t test, *vs*. solvent control group, * p <0.001; *vs*. 45mg/kg CPT-11 group, # p <0.01 | | | | | | | | | | |

### Embodiment 6: The inhibitory effect of various irinotecan preparations on transplanted tumor of human ovarian cancer cells SKOV-3 in nude mice

### Test substances

CPT-1 was a straight-chain PEG-acetic acid-pentapeptides-irinotecan conjugate.

### Drug used as positive control

40 mg/2 ml irinotecan hydrochloride injection (CPT-11) produced by the Aventis Pharma (Dagenham) company, lot number 8UL002-B. The drug was diluted with saline to the desired concentration just before utilization.

### Dose and administration

CPT-1 dose was set as 45 mg/kg (content of irinotecan), which was administered intravenously once a week for three consecutive weeks; the CPT-11 was intravenously administrated once a week at 45 mg/kg and intravenously administered three times a week for three consecutive weeks at 15 mg/kg.

### Animals

BALB/cA nude mice (male, 5-6 weeks old, weighed 18±2 g) were provided by the Shanghai Institute of Materia Medica, Chinese Academy of Sciences. The production certificate number was SCXK (Shanghai) 2008-0017. Animal numbers of each group: 12 mice in negative control group and 6 mice in treatment group.

### Cell lines

Human lung tumor cell strains SKOV-3 were purchased from ATCC. 5 x 10⁶ cells were subcutaneously inoculated in the right armpit of each nude mouse. The transplanted tumors were transferred in nude mice for two generations before utilization.

### Experimental methods

Vigorous tumor tissues were cut into pieces of about 1.5 mm3 and then subcutaneously inoculated to the right armpit nude mice under sterile conditions. The diameters of the subcutaneous transplanted tumors were measured using a vernier caliper. The animals were randomly grouped when the tumors grew to 100-200 mm3.

The animals in treatment group and control group were intravenously administrated with 45 mg/kg CPT-1 and equivalent saline respectively once a week for three consecutive weeks. CPT-11 (15 mg/kg) was intravenously administrated as a positive control drug three times a week for consecutive three weeks. After the administration, the animals were observed consecutively for one week. During the whole experiment, diameters of the transplanted tumors were measured and the animals were weighed two times a week. The tumor volume (TV) was calculated as follows: TV= 1/2 x a x b², wherein, a and b represented length and width respectively. According to the results of measurements, the relative tumor volumes (RTV) were calculated as follows: RTV=Vt/V0. Wherein, V0 were the volumes measured at administration after caging (i.e. d0), Vt were the tumor volumes of every measurements. The index used in evaluation of anti-tumor activity was relative tumor proliferation rate T/C (%) calculated as follows: T/C (%) = (TRTV/CRTV) x 100%, TRTV was RTV of treatment group, CRTV was RTV of negative control group.

### Results and discussion

The results were shown in Table 4. CPT1 (45 mg/kg) administered intravenously once weekly for three consecutive weeks significantly inhibited the growth of subcutaneous transplanted tumors of human ovarian cancer cells SKOV-3 cells in nude mice, the T/C % was 0.53%, and the inhibitory effects were superior to those of CPT-11 which was administered with the same dose and had a T/C % of 96.46%. One week after administration, the volume of the bearing tumor in each mouse were decreased. The positive control CPT-11 intravenously administrated at 15 mg/kg three times a week for three consecutive weeks could also significantly inhibited the growth of subcutaneous transplanted tumors of SKOV-3 cells with a T/C value of 55.69%. During the whole experiment, nude mice in solvent control group and two groups of CPT-11 treatment underwent slight weight loss; only nude mice in CPT-1 group had good growing conditions.

**Table 4. Experimental treatment effects of PEGylated irinotecan on transplanted tumors of human ovarian cancer cells SKOV-3 in nude mice**

| Group | Dose and administration | | Number of animals | | Weight (g) | | TV (mm³, mean±SD) | | RTV (mean±SD ) | T/C (%) |
|---|---|---|---|---|---|---|---|---|---|---|
| | | | At the beginning | At the beginning | At the beginning | At lats | d₀ | d₂₈ | | |
| Solvent control | 0.4ml/mice qw×3w | iv | 12 | 12 | 19.8 | 18.7 | 136±28 | 1770± 588(0) | 13.27± 4.75 | |
| CPT-11 | 15mg/kg, q3w×3w | iv | 6 | 6 | 18.9 | 18.0 | 137±33 | 924± 443(0) | 7.39± 5.22**^{,} | 55.69 |
| CPT-11 | 45mg/kg, qw×3w | iv | 6 | 6 | 19.7 | 18.4 | 136±44 | 1753± 878(0) | 12.80± 3.91 | 96.46 |
| CPT-1 | 45mg/kg, qw×3w | iv | 6 | 6 | 20.7 | 22.7 | 135±35 | 1.27± 1.98(4) | 0.07:1:0.01*^{,#} | 0.53 |

| | | | | | | | | | | |
|---|---|---|---|---|---|---|---|---|---|---|
| Note: t test, *vs*. solvent control group, * p <0.001; *vs*. 45mg/kg CPT-11 group, # p<0.001, the bracketed number indicated the number of animals with tumor regression | | | | | | | | | | |

### Embodiment 9: The inhibitory effect of various irinotecan preparations on transplanted tumor of human colon cancer cells SW-620 in nude mice

### Test substances

CPT-1 was a straight-chain PEG-acetic acid-pentapeptides-irinotecan conjugate.

### Drug used as positive control

40 mg/2 ml irinotecan hydrochloride injection (CPT-11) produced by the Aventis Pharma (Dagenham) company, lot number 8UL002-B. The drug was diluted with saline to the desired concentration just before utilization.

### Dose and administration

CPT-1 dose was set as 45 mg/kg (content of irinotecan), which was administered intravenously once a week for three consecutive weeks; the CPT-11 was intravenously administrated once a week at 45 mg/kg and intravenously administered three times a week for three consecutive weeks at 15 mg/kg.

### Animals

BALB/cA nude mice (male, 5-6 weeks old, weighed 18±2 g) were provided by the Shanghai Institute of Materia Medica, Chinese Academy of Sciences. The production certificate number was SCXK (Shanghai) 2008-0017. Animal numbers of each group: 12 mice in negative control group and 6 mice in treatment group.

### Cell lines

Human lung tumor cell strains SW-620 were purchased from ATCC. 5 x 10⁶ cells were subcutaneously inoculated in the right armpit of each nude mouse. The transplanted tumors were transferred in nude mice for two generations before utilization.

### Experimental methods

Vigorous tumor tissues were cut into pieces of about 1.5 mm3 and then subcutaneously inoculated to the right armpit nude mice under sterile conditions. The diameters of the subcutaneous transplanted tumors were measured using a vernier caliper. The animals were randomly grouped when the tumors grew to 100-200 mm3. The animals in treatment group and control group were intravenously administrated with 45 mg/kg CPT-1 and equivalent saline respectively once a week for three consecutive weeks. CPT-11 (15 mg/kg) was intravenously administrated as a positive control drug three times a week for consecutive three weeks. After the administration, the animals were observed consecutively for one week. During the whole experiment, diameters of the transplanted tumors were measured and the animals were weighed two times a week. The tumor volume (TV) was calculated as follows: TV= 1/2 x a x b², wherein, a and b represented length and width respectively. According to the results of measurements, the relative tumor volumes (RTV) were calculated as follows: RTV=Vt/V0. Wherein, V0 were the volumes measured at administration after caging (i.e. d0), Vt were the tumor volumes of every measurements. The index used in evaluation of anti-tumor activity was relative tumor proliferation rate T/C (%) calculated as follows: T/C (%) = (TRTV/CRTV) x 100%, TRTV was RTV of treatment group, CRTV was RTV of negative control group.

### Results and discussion

The results were shown in Table 5. CPT1 (45 mg/kg) administered intravenously once weekly for three consecutive weeks significantly inhibited the growth of subcutaneous transplanted tumors of human colon tumor cell line SW-620 cells in nude mice, and the inhibitory effects were superior to those of CPT-11 administered with the same dose and regimen. After three weeks of experimental treatment of six tumor-bearing mice in each group, tumors of two tumor-bearing mice in CPT-1 treatment group achieved complete regression without rebound a week after drug discontinuance. The positive control CPT-11 intravenously administrated at 15 mg/kg three times a week for three consecutive weeks could also significantly inhibited the growth of subcutaneous transplanted tumors of SW-620 cells with a T/C value of 0.13%. During the whole experiment, nude mice in each treatment group had good growing conditions and a higher weight gain, compared with the solvent control group.

**Table 5 Experimental treatment effect of PEGylated irinotecan on transplanted tumors of human colon tumor cells SW-620 in nude mice**

| Group | Dose and administration | | Number of animals | | Weight (g) | | TV (mm³, mean±SD) | | RTV (mean±SD) | T/C (%) |
|---|---|---|---|---|---|---|---|---|---|---|
| | | | At the beginning | At last | At the beginning | At last | d₀ | d₂₈ | | |
| Solvent control | 0.4ml/mice qw×3w | i v | 12 | 12 | 19.4 | 19.9 | 132±29 | 1920±590(0) | 15.40±6.40 | |
| CPT-11 | 15mg/kg, q3w×3w | i v | 6 | 6 | 19.7 | 24.2 | 133±33 | 2.09±1.51(0) | 0.02±0.02**^{,} | 0.13 |
| CPT-11 | 45mg/kg, qw×3w | i v | 6 | 6 | 18.8 | 21.8 | 132±25 | 877±324(0) | 6.60±1.56* | 42.86 |
| CPT-1 | 45mg/kg, qw×3w | i v | 6 | 6 | 19.2 | 24.6 | 130±43 | 0.37± 0.29(2) | 0.003±0.002* *^{,#} | 0.02 |

| | | | | | | | | | | |
|---|---|---|---|---|---|---|---|---|---|---|
| Note: t test, *vs*. solvent control group, * p <0.01, ** p <0.00; *vs*. 45mg/kg CPT-11 group, # p <0.001, the bracketed number indicated the number of animals with tumor regression | | | | | | | | | | |

## Claims

1. A PEG-oligopeptide-irinotecan conjugate according to formula (I): 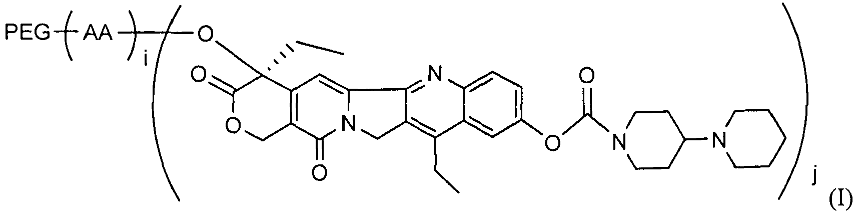 wherein:
PEG represents polyethylene glycol having a straight-chain structure and an average molecular weight of 20,000-40,000 Daltons;
(AA)ᵢ represents an oligopeptide, wherein AA represents the same or different amino acids of the oligopeptide;
i and j can be the same or different;
i is an integer from 2-12 representing the number of amino acids of the oligopeptide; and
j is an integer from 2-12 representing the number of irinotecan compounds connected with the oligopeptide;
wherein the amino acids are glutamic acid and glycine and the oligopeptide consists of 2 glutamic acid and 3 glycine residues, or the oligopeptide consists of 3 glutamic acid and 4 glycine residues.

2. The conjugate according to claim 1, wherein the PEG is a straight-chain methoxy-PEG with a structure of: 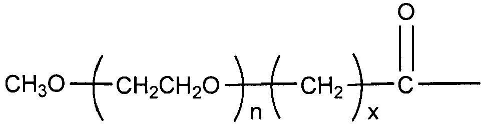 wherein:
n is an integer from 400-500; and
x is an integer from 0-6, preferably x is 1.

3. A pharmaceutical composition comprising the conjugate according to claim 1 or claim 2 and a pharmaceutically acceptable carrier or excipient.

4. The pharmaceutical composition according to claim 3, wherein the pharmaceutical composition is in a dosage form of tablets, suppositories, pellets, soft and hard gelatin capsules, powders, solutions, suspensions or aerosols.

5. The pharmaceutical composition according to claim 4, wherein the pharmaceutical composition has anti-cancer activities including an inhibitory property against colon cancer, ovarian cancer or lung cancer.

## Patentansprüche

1. PEG-Oligopeptid-Irinotecan-Konjugat gemäß Formel (I): 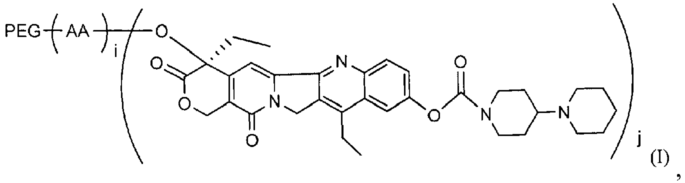 wobei:
PEG Polyethylenglycol darstellt, das eine geradkettige Struktur und ein durchschnittliches Molekulargewicht von 20 000-40 000 Dalton aufweist;
(AA)ᵢ ein Oligopeptid darstellt, wobei AA die gleichen oder unterschiedliche Aminosäuren des Oligopeptids darstellt;
i und j gleich oder unterschiedlich sein können;
i eine ganze Zahl von 2-12 ist, die die Anzahl an Aminosäuren des Oligopeptids darstellt; und
j eine ganze Zahl von 2-12 ist, die die Anzahl an Irinotecanverbindungen darstellt, die mit dem Oligopeptid verbunden sind;
wobei die Aminosäuren Glutaminsäure und Glycin sind und das Oligopeptid aus 2 Glutaminsäure- und 3 Glycinresten besteht oder das Oligopeptid aus 3 Glutaminsäure- und 4 Glycinresten besteht.

2. Konjugat nach Anspruch 1, wobei das PEG ein geradkettiges Methoxy-PEG mit einer folgenden Struktur ist: 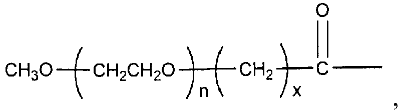 wobei:
n eine ganze Zahl von 400-500 ist; und
x eine ganze Zahl von 0-6 ist, bevorzugt x 1 ist.

3. Pharmazeutische Zusammensetzung, die das Konjugat nach Anspruch 1 oder Anspruch 2 und einen pharmazeutisch verträglichen Träger oder Hilfsstoff umfasst.

4. Pharmazeutische Zusammensetzung nach Anspruch 3, wobei die pharmazeutische Zusammensetzung in einer Dosierungsform von Folgenden vorliegt: Tabletten, Suppositorien, Pellets, Weich- und Hartgelatinekapseln, Pulvern, Lösungen, Suspensionen oder Aerosolen.

5. Pharmazeutische Zusammensetzung nach Anspruch 4, wobei die pharmazeutische Zusammensetzung Antikrebsaktivitäten aufweist, die eine inhibitorische Eigenschaft gegen Dickdarmkrebs, Eierstockkrebs oder Lungenkrebs einschließen.

## Revendications

1. Conjugué de PEG-oligopeptide-irinotécan répondant à la formule (I) : 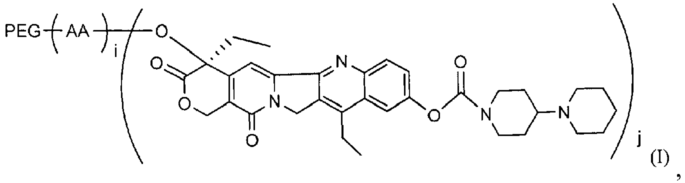 dans lequel :
PEG représente un polyéthylène glycol ayant une structure à chaîne linéaire et un poids moléculaire moyen de 20 000-40 000 Daltons ;
(AA)ᵢ représente un oligopeptide, où AA représente des acides aminés identiques ou
différents de l'oligopeptide ;
i et j peuvent être identiques ou différents ;
i est un nombre entier valant 2-12 et représentant le nombre d'acides aminés de l'oligopeptide ; et
j est un nombre entier valant 2-12 et représentant le nombre de composés d'irinotécan reliés à l'oligopeptide ;
où les acides aminés sont l'acide glutamique et la glycine, et l'oligopeptide est constitué de 2 résidus d'acide glutamique et de 3 résidus de glycine, ou l'oligopeptide est constitué de 3 résidus d'acide glutamique et de 4 résidus de glycine.

2. Conjugué selon la revendication 1, dans lequel le PEG est un PEG méthoxylé à chaîne linéaire répondant à la structure : 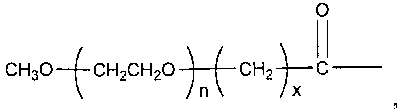 dans lequel :
n est un nombre entier valant 400-500 ; et
x est un nombre entier valant 0-6, préférablement x vaut 1.

3. Composition pharmaceutique comprenant le conjugué selon la revendication 1 ou la revendication 2, et un véhicule ou excipient pharmaceutiquement acceptable.

4. Composition pharmaceutique selon la revendication 3, où la composition pharmaceutique se trouve sous une forme de dosage en comprimés, suppositoires, pastilles, capsules de gélatine molle et dure, poudres, solutions, suspensions ou aérosols.

5. Composition pharmaceutique selon la revendication 4, où la composition pharmaceutique possède des activités anticancéreuses, y compris une propriété inhibitrice contre le cancer du côlon, le cancer de l'ovaire ou le cancer du poumon.
